(19) Europäisches Patentamt
European Patent Office
Office européen des brevets

(11) Veröffentlichungsnummer: **0 029 804**
**B1**

(12) # EUROPÄISCHE PATENTSCHRIFT

(45) Veröffentlichungstag der Patentschrift: **16.01.85**

(51) Int. Cl.⁴: **D 06 P 3/60**, D 06 P 3/85, D 06 L 3/12, C 07 D 201/18, C 07 D 201/14

(21) Anmeldenummer: **80810327.9**

(22) Anmeldetag: **30.10.80**

(54) **Verfahren zum Bedrucken oder Klotzfärben von Textilmaterial aus Cellulosefasern in Mischung mit synthetischen Fasern sowie Druckpasten oder Klotzflotten zur Durchführung dieses Verfahrens.**

(30) Priorität: **05.11.79 CH 9908/79**

(43) Veröffentlichungstag der Anmeldung: **03.06.81 Patentblatt 81/22**

(45) Bekanntmachung des Hinweises auf die Patenterteilung: **16.01.85 Patentblatt 85/3**

(84) Benannte Vertragsstaaten: **CH DE FR GB IT LI**

(56) Entgegenhaltungen:
**DE - A - 2 554 923**
**DE - A - 2 619 023**
**DE - A - 2 635 650**
**DE - A - 2 700 150**
**DE - A - 2 829 299**
**FR - A - 2 118 186**
**FR - A - 2 270 287**
**GB - A - 1 249 896**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Postfach, CH-4002 Basel (CH)**

(72) Erfinder: **Koci, Zdenek, Rebgasse 39, CH-4102 Binningen (CH)**
Erfinder: **Schaub, Andres, Chillweg 6, CH-4105 Biel-Benken (CH)**

**EP 0 029 804 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zum Bedrucken oder Klotzfärben von Textilmaterial aus Cellulosefasern in Mischung mit synthetischen Fasern sowie die Druckpasten oder Klotzflotten zur Durchführung dieses Verfahrens.

Färbt man Mischfasern aus Cellulose und synthetischem Material mit Dispersionsfarbstoffen, so wird das natürliche Material nicht gefärbt, sondern allenfalls angeschmutzt, während man beim Färben mit Farbstoffen, die zum Färben des natürlichen Materials gut geeignet sind, wie z.B. Reaktiv- oder Direktfarbstoffen, nur eine ungenügende Anfärbung des synthetischen Materials erhält.

Zum farbigen Bedrucken von Mischfasern der oben genannten Art wird daher üblicherweise eine Druckpaste verwendet, welche ein Farbstoffgemisch aus den für die einzelnen Bestandteile der Mischfasern jeweils geeigneten Farbstoffen enthält, wobei jedoch die Bestandteile der Mischfasern nur selten in genau der gleichen Nuance gefärbt werden können.

Aus der CA-A-832 343 ist ein Verfahren zum Bedrucken von Mischfasern aus Cellulose und synthetischem Material bekannt, in welchem beide Bestandteile der Mischfasern mit dem gleichen Dispersionsfarbstoff gefärbt werden, wobei eine Druckpaste verwendet wird, welche Dispersionsfarbstoff, Wasser und ein Polyalkylenglykol als Lösungsmittel für den Farbstoff enthält.

Die Verwendung dieser Lösungsmittel für den Farbstoff ergibt jedoch bei vielen Farbstoffen, insbesondere bei den sogenannten organischen Pigmentfarbstoffen, keine befriedigenden Ergebnisse, da das Lösungsvermögen der verwendeten Polyalkylenglykole nicht ausreicht. Deshalb ist dieses Verfahren nur mit speziellen Dispersionsfarbstoffen durchführbar.

Ausserdem sind die genannten Lösungsmittel, insbesondere die niedermolekularen, hygroskopisch, so dass die mit der Druckpaste bzw. Klotzflotte imprägnierten und getrockneten Textilmaterialien leicht feucht werden können und infolgedessen vor der Fixierung zum Abschmieren bzw. Abflecken neigen.

Ferner sind in der DE-A-2 619 023 und der DE-A-2 635 650 Verfahren zum Färben von cellulosehaltigen Mischgeweben, insbesondere Baumwolle/Polyester-Mischungen beschrieben. Während in ersterer als Hilfsmittel neben grenzflächenaktiven Substanzen, organischen Lösungsmitteln und Verdickern auch begrenzt wasserlösliche Carrier verwendet werden, so können diese im Verfahren gemäss letzterer weggelassen werden, für den Fall, dass es sich bei den organischen Lösungsmitteln und solche handelt, die wasserlöslich sind. Als geeignete organische Lösungsmittel sind in der DE-A-2 635 650 u.a. Caprolactam und niedere Mono- und Dialkyläther des Triäthylenglykols genannt. Niedermolekulare Glykoläther haben jedoch den Nachteil, dass sie beim Fixieren zumindest teilweise verdampfen und so die Abluft belasten. Zudem enthalten die in den Beispielen der genannten Offenlegungsschriften angegebenen Klotzflotten und Druckpasten erhebliche Mengen an oberflächenaktiven Substanzen, die wie die anderen Hilfsmittel bei der Fertigstellung des gefärbten Materials wieder ausgewaschen werden müssen und so in das Abwasser gelangen.

Der vorliegenden Erfindung lag die Aufgabe zugrunde, ein Verfahren zum Bedrucken und Klotzfärben von Textilmaterial aus Cellulosefasern oder Gemischen derselben mit synthetischen Fasern unter Verwendung von in Wasser schwer löslichen oder unlöslichen Farbstoffen zu entwickeln, welches die oben genannten Nachteile nicht aufweist.

Diese Aufgabe wird erfindungsgemäss dadurch gelöst, dass man ausgewählte Hilfsmittel verwendet.

Die vorliegende Erfindung betrifft somit ein Verfahren zum Bedrucken oder Klotzfärben von Textilmaterial aus Cellulosefasern in Mischung mit synthetischen Fasern mit wässrigen Druckpasten oder Klotzflotten, indem man die Fasern entweder gleichzeitig oder nacheinander mit einer wässrigen Dispersion mindestens eines in Wasser schwer löslichen oder unlöslichen Farbstoffes, welche als weitere Zusätze gegebenenfalls Antioxidantien, Netzmittel, Antischaummittel, Weichmachungsmittel, Zusätze zum Flammfestausrüsten, schmutz-, wasser- und ölabweisende Mittel, natürliche oder synthetische Verdicker und/oder eine Säure enthält, und einer wässrigen Lösung eines Hilfsmittels imprägniert und anschliessend den Druck oder die Färbung einer Hitzebehandlung unterwirft, dadurch gekennzeichnet, dass man als Hilfsmittel einen Komplex aus einem Lactam der Formel I

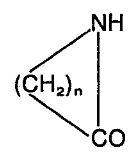 (I)

worin n eine ganze Zahl von 3 bis 6 bedeutet, und einem Polyalkylenglykol der Formel II

$$HO-(\underset{R}{\underset{|}{C}}H-CH_2-O-)_yH \qquad (II)$$

verwendet, worin R Wasserstoff oder eine Methylgruppe und y eine ganze Zahl von 5 bis 10 bedeutet.

Als Lactame der Formel I kommen beispielsweise Butyrolactam, Valerolactam, Caprolactam oder Oenantholactam, vor allem ε-Caprolactam in Betracht.

Bevorzugt zur Anwendung gelangen die 1 : 1 Molkomplexe, insbesondere die diejenigen aus ε-Caprolactam und einem Polyäthylenglykol oder Polypropylenglykol der oben angegebenen Formel II.

Die erfindungsgemäss verwendbaren Hilfsmittel wirken als Farbstofflösungsmittel. Sie werden nach an sich bekannten Methoden hergestellt. Die 1 : 1 Molkomplexe aus Lactamen und Polyalkylenglykolen erhält man z.B., indem man die Komponenten in einem geeigneten organischen Lösungsmittel oder Wasser löst, gegebenenfalls unter Erwärmen, und anschliessend das organische Lösungsmittel oder Wasser abdestilliert oder indem man beide Komponenten unter Rühren miteinander vermischt bis eine klare Lösung entsteht, wobei man eventuell erwärmt, falls eine oder beide Komponenten bei Raumtemperatur fest sind.

Es ist vorteilhaft, den Komplexen aus Lactam und Polyalkylenglykol, 0,1 bis 5 Gew.-%, insbesondere 0,5 bis 1 Gew.-%, bezogen auf das Gewicht des Komplexes, an Antioxidantien zuzusetzen.

Als Antioxidantien kommen z.B. in Frage: durch sterisch hindernde Gruppen substituierte Phenole, aromatische Amine und Hydrochinone, wie Di-tert.-butyl-hydroxytoluol,
Tert.-butylhydroxyanisol,
2,5-Di-tert.-butylhydrochinon,
3,5-Di.-tert.-butylbrenzkatechin,
ferner Alkylidenbisphenole, wie
4,4′-Methylen-bis-(2,6-di-tert.-butylphenol)
sowie hydroxylierte Thiodiphenyläther,
O-, N- und S-Benzylverbindungen,
hydroxybenzylierte Malonester,
Hydroxybenzyl-Aromaten,
s-Triazinverbindungen,
Amide der β-(3,5-Di-tert.-butyl-4-hydroxyphenyl)-propionsäure,
Ester der β-(3,5-Di-tert.-butyl-4-hydroxyphenyl)-propionsäure,
Ester der β-(5-tert.-Butyl-4-hydroxy-3-methylphenyl)-propionsäure,
Ester der 3,5-Di-tert.-butyl-4-hydroxyphenylessigsäure,
Aclyaminophenole, Benzylphosphonate
oder vorzugsweise Gallussäurepropylester.

Der Zusatz von Antioxidantien vermindert die oxydative thermische Zersetzung, besonders der Polyalkylenglykol-Komponente.

Diese thermische Zersetzung ist besonders hoch bei Temperaturen über 200 °C und verursacht neben der Farbausbeuteverschlechterung (wegen einer erniedrigten Konzentration des Farbstofflösungsmittels im Gewebe) auch eine erhöhte Abluft- evtl. Apparat-Verschmutzung.

Besonders bevorzugte Hilfsmittel stellen die 1 : 1 Molkomplexe aus ε-Caprolactam und Polypropylenglykol mit einem Molekulargewicht zwischen 300 und 500, vorzugsweise ca. 400, dar, welche 0,5 bis 1 Gew.-%, bezogen auf das Gewicht des 1 : 1 Molkomplexes, Gallussäurepropylester enthalten.

Das erfindungsgemäss verwendbare Hilfsmittel wird vorzugsweise in einer Menge von 5–30 Gew.-%, insbesondere 10–20 Gew.-%, bezogen auf das Gewicht der Druckpaste oder Klotzflotte, eingesetzt. Es wird vorzugsweise der Druckpaste oder Klotzflotte zugesetzt, bevor diese auf das zu färbende oder bedruckende Textilmaterial aufgebracht wird. Es ist aber auch möglich, das Hilfsmittel gesondert vor oder nach der Imprägnierung des Textilmaterials mit der Druckpaste oder Klotzflotte auf dieses aufzubringen, z.B. durch Bedrucken, Foulardieren, Pflatschen, Eintauchen oder Besprühen des Textilmaterials mit einer wässrigen Lösung des Hilfsmittels.

Geeignete Farbstoffe für das erfindungsgemässe Verfahren müssen in Wasser unlöslich oder schwer löslich sein. Es handelt sich z.B. um die im «Colour Index» unter den Bezeichnungen disperse dyes, vat dyes, solvent dyes, azoic dyes, oder pigments beschriebenen Farbstoffe. Diese können verschiedenen Klassen angehören, beispielsweise kommen in Frage: Nitrofarbstoffe, Aminoketonfarbstoffe, Ketoniminfarbstoffe, Methinfarbstoffe, Nitrodiphenylaminfarbstoffe, Chinolinfarbstoffe, Aminonaphthochinonfarbstoffe, Cumarinfarbstoffe und insbesondere Antrachinonfarbstoffe und Azofarbstoffe, wie Monoazo- und Disazofarbstoffe.

Als Küpenfarbstoffe kommen insbesondere höher annelierte und heterocyclische Benzo- und Naphtochinone, Schwefelfarbstoffe sowie vor allem anthrachinoide und indigoide Farbstoffe in Betracht.

Unter Farbstoffen sollen auch optische Aufheller verstanden werden. Es kommen z.B. in Wasser unlösliche bis schwer lösliche Aufheller der folgenden Verbindungsklassen in Frage: Stilbene, Cumarine, Benzocumarine, Pyrene, Pyrazine, Oxazine, Mono- oder Dibenzoxazolyl- oder -imidazolylverbindungen, Aryltriazol- und v-Triazol-Derivate sowie Naphthalsäureimide.

Die Druckpaste oder Klotzflotte zur Durchführung des erfindungsgemässen Verfahrens enthält in der Regel 45–95 Gew.-% Wasser, 0,1–10 Gew.-% des Farbstoffes, 5–30 Gew.-% Lactam/Polyalkylenglykol-Komplex und 0,1–15 Gew.-%, bezogen auf das Gesamtgewicht, weitere Zusätze, und zwar die bereits erwähnten Antioxidantien sowie Netzmittel, Antischaummittel oder die Eigenschaften des Textilmaterials beeinflussende Mittel, wie z.B. Weichmachungsmittel, Zusätze zum Flammfestausrüsten oder schmutz-, wasser- und ölabweisende Mittel, vor allem aber natürliche oder synthetische Verdicker und/oder eine Säure, wie z.B. Essigsäure oder Citronensäure. Vorzugsweise enthält die Druckpaste oder Klotzflotte 60–80 Gew.-% Wasser, 1–8 Gew.-% des Farbstoffes, 10–25 Gew.-% Lactam/Polyalkylenglykol-Komplex und 0,2–8 Gew.-% weitere Zusätze.

Geeignete natürliche Verdickungsmittel sind beispielsweise Kernmehläther, Stärkeäther, Alginate, Stärke, Tragant, Carboxymethylcellulose und Cellulsoeäther. Als synthetische Verdickungsmittel kommen beispielsweise hochmolekulare Mono- oder Copolymerisate von Acrylsäure, Methacrylsäure, Maleinsäure mit äthylenisch ungesättigten Comonomeren, wie Äthylen, Butadien, Hydroxyalkylacrylate, Divinyldioxan und Divinylbenzol, in Form von wasserlöslichen Alkali-, Ammonium- oder Amin-Salzen in Frage.

Der Gehalt der Klotzflotte an Verdicker liegt bei etwa 0,1–5 Gew.-%, die Druckpasten enthalten ca. 0,2–10 Gew.-%, je nach gewünschter Viskosität.

Nach dem erfindungsgemässen Verfahren kann Textilmaterial aus Cellulosefasern in Mischung mit synthetischen Fasern gefärbt oder bedruckt

werden, wobei als Cellulosefasern solche aus natürlicher und regenerierter Cellulose in Betracht kommen, wie z.B. Hanf, Leine, Jute, Viskose-Seide, Zellwolle oder insbesondere Baumwolle.

Als synthetische organische Materialien kommen z.B. in Frage: Fasermaterialien aus synthetischem Polyamid, wie Kondensationsprodukte aus Hexamethylendiamin und Adipinsäure (Polyamid 6,6) oder Sebacinsäure (Polyamid 6,10), ferner Mischkondensationsprodukte z.B. aus Hexamethylendiamin, Adipinsäure und ε-Caprolactam (Polyamid 6,6/6), ausserdem Polymerisationsprodukte aus ε-Caprolactam oder aus ω-Aminoundecansäure. Ferner kommt Polyestermaterial in Betracht, z.B.: lineare hochmolekulare Ester aromatischer Polycarbonsäuren mit polyfunktionellen Alkoholen, beispielsweise solche aus Terephthalsäure und Äthylenglykol oder Dimethylolcyclohexan, sowie Mischpolymere aus Terephthalsäure und Isophthalsäure und Äthylenglykol.

Schliesslich sind auch noch Cellulose (2 ½)-Acetat- und Cellulosetriacetatfasern als synthetisches Fasermaterial geeignet.

Vorzugsweise wird nach dem erfindungsgemässen Verfahren Textilmaterial aus Fasermischungen gefärbt oder bedruckt, welche aus 2 Komponenten bestehen, insbesondere Fasermischungen aus Polyester/Baumwolle; es lassen sich jedoch auch Fasermischungen verwenden, welche neben Cellulose noch 2 oder mehrere der oben aufgeführten Fasermaterialien enthalten.

Das Textilmaterial kann z.B. als Gewebe, Maschenware, wie z.B. Strickware oder Gewirke, oder als Vlies vorliegen.

Das Textilmaterial wird in bekannter Art und Weise mit der Druckpaste oder Klotzflotte bedruckt oder foulardiert und anschliessend getrocknet. Danach werden die Farbstoffe auf dem Mischgewebe fixiert, beispielsweise durch Erhitzen während 30–120 Sekunden auf 150–225 °C, vorzugsweise während 60 Sekunden auf 210–215 °C, oder durch Dämpfen, z.B. bei Normaldruck mit überhitztem Dampf von 170–200 °C während 3–12, vorzugsweise 5–8 Minuten oder mit Wasserdampf von 1,5 Bar Überdruck während 15–30 Minuten.

Nach dem Fixieren wird wie üblich fertiggestellt. Man erhält nach diesem Verfahren egale Ton-in-Ton Drucke oder Färbungen mit guter Farbstoffausbeute.

Im Vergleich zur Verwendung von Polyalkylenglykolen besitzen die im vorliegenden Verfahren verwendeten Komplexe von Lactamen mit Polyalkylenglykolen ein erhöhtes Lösungsvermögen für wasserunlösliche Farbstoffe, besonders für Pigmentfarbstoffe, was zu einer besseren Farbausbeute nach der Fixierung der Farbstoffe führt, insbesondere auf dem Cellulosematerial. Aber auch auf dem synthetischen Material ist die Farbausbeute höher als wenn ohne Hilfsmittel oder nur mit Polyalkylenglykolen gefärbt wird. Ausserdem sind die Komplexe weniger hygroskopisch als die einzelnen Komponenten, so dass die Drucke bzw. Färbungen vor der Fixierung weniger leicht feucht werden und deshalb nicht zum Abschmieren bzw. Abflecken neigen. Schliesslich sind die Komplexe besser biologisch abbaubar, da die Lactam-Komponente besser abbaubar ist als die Polyalkylenglykol-Komponente.

Die nachfolgenden Beispiele dienen zur Erläuterung des erfindungsgemässen Verfahrens. Teile sind Gewichtsteile und die Temperaturen sind in Celsiusgraden angegeben.

Beispiel 1:

Ein Mischgewebe, bestehend aus 50% Baumwolle und 50% Polyester mit einem Quadratmetergewicht von 120 g wird im Rouleaudruck mit einer Druckpaste bedruckt, die aus

15 Teilen des Farbstoffes Pigmentgelb 1 (CI 11 680), als 20%ige flüssige Handelsform,
500 Teilen einer 8%igen Na-Alginatverdickung,
150 Teilen 1 : 1 Molkomplex von Caprolactam mit Polyäthylenglykol (Mol.-Gew. 400)
2 Teilen Citronensäure und
333 Teilen Wasser

besteht. Anschliessend wird bei 110° getrocknet und während 60 Sekunden bei 215° in Heissluft fixiert. Danach wird das Gewebe mit Wasser gespült, mit einer heissen Seifenlösung behandelt und nochmals mit Wasser gespült. Es resultiert ein egaler Druck mit guten Echtheiten.

Wird der Druck ohne Caprolactam Polyäthylenglykol-Komplex ausgeführt, so ist der Farbausfall deutlich heller und unruhig.

Beispiel 2:

Ein Baumwoll-Polyester-Mischgewebe aus je 50 Teilen Baumwolle und Polyester wird an einem Foulard getränkt und auf 90% Flottenaufnahme abgepresst mit einer Flotte, bestehend aus

100 Teilen 1 : 1 Molkomplex aus Caprolactam und Polyäthylenglykol (Mol.-Gew. 300) und
900 Teilen Wasser.

Anschliessend wird bei 100° getrocknet und bedruckt mit einer Druckfarbe, die aus

80 Teilen des Farbstoffes Pigmentrot 112 (CI 12 370), als 20%ige flüssige Handelsform,
500 Teilen Na-Alginatverdickung 127%ig und
420 Teilen Wasser

besteht.

Nach dem Trocknen wird während 60 Sekunden bei 215 °C fixiert.

Der nichtfixierte Farbstoff lässt sich durch kaltes und heisses Spülen entfernen.

Der resultierende rote Druck weist gute Echtheiten und eine gute Ton-in-Ton-Färbung der beiden Faseranteile auf.

Beispiel 3:

Arbeitet man wie im Beispiel 2 beschrieben, verwendet jedoch anstelle von 100 Teilen des 1 : 1 Molkomplexes von Caprolactam und Polyäthylenglykol (Mol.-Gew. 300) 100 Teile des gleichen Komplexes, der zusätzlich 1% Gallussäure-Propylester als Antioxidans enthält, so resultiert eine etwas tiefere Färbung und eine verminderte Rauchentwicklung aus dem fixierten Gewebe als bei Verwendung des Komplexes ohne Antioxidant.

Die stabilisierende Wirkung des Antioxidans zeigt sich auch, wenn man den obengenannten Molkomplex sowie den gleichen Komplex, der 1% Gallussäure-Propylester enthält, mit einer Aufheizgeschwindigkeit von 20 °C/Min. auf 225 °C erhitzt. Der Gewichtsverlust bei dem unstabilisierten Komplex beträgt 42,5%, bei dem stabilisierten Komplex dagegen nur 30,9%.

Beispiel 4:

Ein Mischgewebe aus 67 Teilen Polyester und 33 Teilen Baumwolle wird mit einer wässerigen Flotte geklotzt, die aus

60 Teilen Pigmentrot 7 (CI 12 420), als 20%ige flüssige Handelsform,
1 Teil hochviskosem Natriumalginat,
1 Teil Essigsäure, 80%ig,
150 Teilen 1 : 1 Molkomplex von Caprolactam und Polypropylenglykol (Mol.-Gew. 400), stabilisiert mit 1% Thiodiäthylenglykol-β-[3,5-di-t.-butyl-4-hydroxyphenyl]propionat als Antioxidans und
788 Teilen Wasser

besteht, und auf eine Flottenaufnahme von 65% des Fasergewichtes abgequetscht. Das Gewebe wird anschliessend während 2 Minuten bei 120 °C getrocknet und 1 Minute bei 210 °C thermosoliert. Dann wird das Gewebe 10 Minuten lang mit einer Flotte, welche 1 g/l eines anionischen Alkyläthersulfates und 1 g/l Soda calc. enthält, bei 98 °C geseift und zuletzt gespült. Man erhält eine gleichmässig brillante rote Färbung auf beiden Faseranteilen mit guten Echtheiten.

Beispiel 5:

Ein Mischgewebe aus 50 Teilen Polyester und 50 Teilen Baumwolle wird mit einer wässerigen Flotte geklotzt, die aus

60 Teilen Pigmentgelb (CI 11 680), als 20%ige flüssige Handelsform,
1 Teil hochviskosem Natriumalginat,
1 Teil Essigsäure, 80%ig,
150 Teilen 1 : 1 Molkomplex von Valerolactam mit Polyäthylenglykol (Mol.-Gew. 300)
788 Teilen Wasser

besteht, und auf eine Flottenaufnahme von 70% des Fasergewichtes abgequetscht. Das Gewebe wird anschliessend 2 Minuten bei 120 °C getrocknet und 1 Minute bei 210 °C thermosoliert. Dann wird das Gewebe 10 Minuten lang mit einer Flotte, welche 1 g/l eines anionischen Alkyläthersulfats und 1 g/l Soda calc. enthält, bei 98 °C geseift und zuletzt gespült. Man erhält eine gleichmässig brillante gelbe Färbung auf beiden Faseranteilen mit guten Echtheiten.

Auf gleiche Art und Weise werden auch ein Gewebe aus 100%iger Baumwolle und ein Gewebe aus 100% Polyester gefärbt. Das Gewebe aus Baumwolle wird genauso gewaschen wie das Mischgewebe Polyester/Baumwolle und das Gewebe aus 100%igem Polyester wird 15 Minuten bei 70 °C mit einer Flotte, welche 5 ml/l NaOH (Dichte 1,33), 3 g/l Hydrosulfit und 1 g/l eines Alkylaminpolyglykoläthers enthält, gewaschen.

Man erhält auf beiden Fasermaterialien eine brillante gelbe Färbung mit guten Echtheiten.

Beispiel 6:

Arbeitet man wie im Beispiel 5 beschrieben, verwendet jedoch als Farbstoff Küpengelb 2 (CI 67 300) oder den gelben Farbstoff der Formel

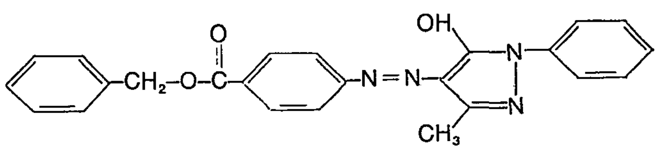

so erhält man gleich gute Ton-in-Ton-Färbungen.

Beispiele 7–13:

Ein Polyester-Baumwoll-Mischgewebe (67 : 33) wird an einem Foulard getränkt und auf 70% Flottenaufnahme abgepresst mit einer Flotte bestehend aus

6 Teilen eines in der folgenden Tabelle aufgeführten Farbstoffes,
10 Teilen Na-Alginatverdickung 1 : 100
2 Teilen Netzmittel (wässrige Mischung, enthaltend Kokosfettsäurediäthanolamid und äthoxyliertes Nonylphenol)
0,1 Teilen Essigsäure 80%
15 Teilen 1 : 1 Molkomplex aus Caprolactam und Polypropylenglykol (Mol.-Gew. ca. 400) und
66,9 Teilen Wasser.

Anschliessend wird 2 Minuten bei 120 °C getrocknet und während 1 Minute bei 210 °C thermosoliert. Danach wäscht man 10 Minuten bei Kochtemperatur mit einer Flotte, welche pro Liter 1 g Nonylphenoldiglykoläthersulfat und 1 g Soda enthält.

Man erhält gleichmässige Färbungen auf beiden Faseranteilen in den angegebenen Farben mit guten Echtheiten.

| Bsp. | Farbstoff | Nuance auf PES/CO |
|---|---|---|
| 7 | Pigment Red 4; CI 12 085 | rot |
| 8 | 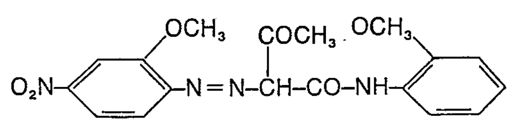 | gelb |

| Bsp. | Farbstoff | Nuance auf PES/CO |
|---|---|---|
| 9 | Pigment Red 7; CI 12 420 | rot |
| 10 | 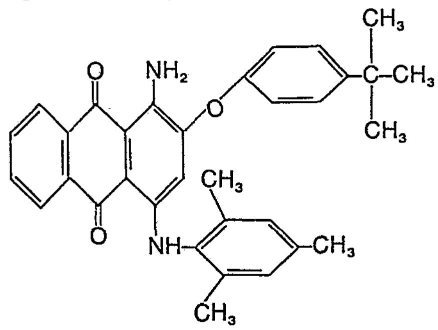 | violett |
| 11 | 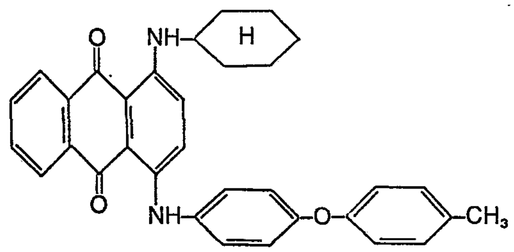 | blau |
| 12 | 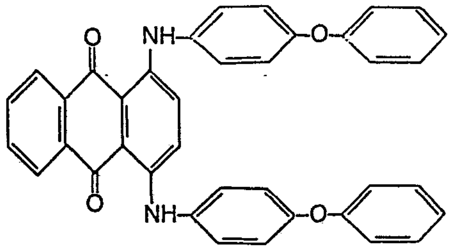 | grün |
| 13 | 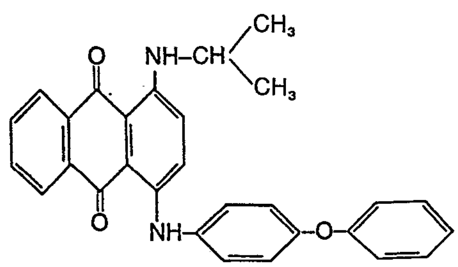 | blau |

Arbeitet man wie in den Beispielen 7–13 beschrieben, verwendet jedoch eine Foulardflotte ohne 1 : 1 Molkomplex aus Caprolactam und Polypropylenglykol, so erhält man sehr unegale Färbungen, da der Baumwollanteil praktisch nicht gefärbt ist.

**Patentansprüche**

1. Verfahren zum Bedrucken oder Klotzfärben von Textilmaterial aus Cellulosefasern in Mischung mit synthetischen Fasern mit wässrigen Druckpasten oder Klotzflotten, indem man das Textilmaterial entweder gleichzeitig oder nacheinander mit einer wässrigen Dispersion mindestens eines in Wasser schwer löslichen oder unlöslichen Farbstoffes, welche als weitere Zusätze gegebenenfalls Antioxidantien, Netzmittel, Antischaummittel, Weichmachungsmittel, Zusätze zum Flammfestausrüsten, schmutz-, wasser- und ölabweisende Mittel, natürliche oder synthetische Verdicker und/oder eine Säure enthält, und einer wässrigen Lösung eines Hilfsmittels imprägniert und anschliessend den Druck oder die Färbung einer Hitzebehandlung unterwirft, dadurch gekennzeichnet, dass man als Hilfsmittel einen Komplex aus einem Lactam der Formel I

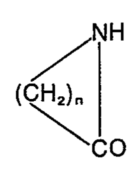 (I)

worin n eine ganze Zahl von 3 bis 6 bedeutet, und einem Polyalkylenglykol der Formel II

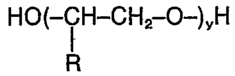 (II)

verwendet, worin R Wasserstoff oder eine Methylgruppe und y eine ganze Zahl von 5 bis 10 bedeutet.

2. Verfahren gemäss Anspruch 1, dadurch gekennzeichnet, dass als Hilfsmittel ein 1 : 1 Molkomplex aus ε-Caprolactam und einem Polyalkylenglykol der Formel II verwendet wird, wobei R und y die im Anspruch 1 angegebene Bedeutung haben.

3. Verfahren gemäss Anspruch 1, dadurch gekennzeichnet, dass man 0,1–5 Gew.-%, vorzugsweise 0,5–1 Gew.-%, bezogen auf den Lactam/Polyalkylenglykol-Komplex, an Antioxidantien verwendet.

4. Verfahren gemäss Anspruch 3, dadurch gekennzeichnet, dass man als Antioxidans Gallussäure-Propylester einsetzt.

5. Verfahren gemäss Ansprüchen 1 bis 4, dadurch gekennzeichnet, dass man den Lactam/Polyalkylenglykol-Komplex in einer Menge von 5–30 Gew.-%, vorzugsweise 10–20 Gew.-%, bezogen auf das Gewicht der Druckpaste oder Klotzflotte, verwendet.

6. Verfahren gemäss Anspruch 1, dadurch gekennzeichnet, dass man den 1 : 1 Molkomplex aus ε-Caprolactam und Polypropylenglykol mit einem Molekulargewicht zwischen 300 und 500, vorzugsweise ca. 400, enthaltend 0,5–1 Gew.-% Gallussäure-Propylester, verwendet.

7. Verfahren gemäss Ansprüchen 1 bis 6, dadurch gekennzeichnet, dass das Hilfsmittel in der wässrigen Druckpaste oder Klotzflotte enthalten ist.

8. Verfahren gemäss Anspruch 1, dadurch gekennzeichnet, dass man Textilmaterial aus Cellulosefasern in Mischung mit Polyesterfasern färbt oder bedruckt.

9. Verfahren gemäss Anspruch 1, dadurch gekennzeichnet, dass man das imprägnierte oder bedruckte Material bei 80–130 °C trocknet und anschliessend einer Hitzeeinwirkung von 150–225 °C unterwirft.

10. Druckpaste oder Klotzflotte zur Durchführung des Verfahrens gemäss Ansprüchen 1 und 7, enthaltend Wasser, einen in Wasser schwer löslichen oder unlöslichen Farbstoff, ein Hilfsmittel sowie als weitere Zusätze gegebenenfalls Antioxidantien, Netzmittel, Antischaummittel, Weichmachungsmittel, Zusätze zum Flammfestausrüsten, schmutz-, wasser- und ölabweisende Mittel, natürliche oder synthetische Verdicker und/oder eine Säure, dadurch gekennzeichnet, dass sie als Hilfsmittel einem Komplex aus einem Lactam der Formel I

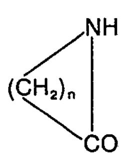 (I)

worin n eine ganze Zahl von 3–6 bedeutet, und einem Polyalkylenglykol der Formel II

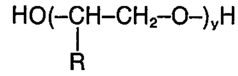 (II)

verwendet, worin R Wasserstoff oder eine Methylgruppe und y eine ganze Zahl von 5–10 bedeutet.

11. Druckpaste oder Klotzflotte gemäss Anspruch 10, dadurch gekennzeichnet, dass sie als Hilfsmittel einen 1 : 1 Molkomplex aus ε-Caprolactam und einem Polyalkylenglykol der Formel II enthält, wobei R und y die in Anspruch 1 angegebene Bedeutung haben.

12. Druckpaste oder Klotzflotte gemäss Ansprüchen 10 und 11, dadurch gekennzeichnet, dass sie 45–95 Gew.-% Wasser, 0,1–10 Gew.-% des Farbstoffes, 5–30 Gew.-% Lactam/Polyalkylenglykol-Komplex und 0,1–15 Gew.-%, bezogen auf das Gesamtgewicht, weitere Zusätze enthält.

13. Druckpaste oder Klotzflotte gemäss Anspruch 12, dadurch gekennzeichnet, dass sie 60–80 Gew.-% Wasser, 1–8 Gew.-% des Farbstoffes, 10–25 Gew.-% Lactam/Polyalkylenglykol-Komplex und 0,2–8 Gew.-% weitere Zusätze enthält.

14. Druckpaste oder Klotzflotte gemäss Ansprüchen 10 bis 13, dadurch gekennzeichnet, dass sie als weitere Zusätze Verdicker und/oder ein Antioxidans enthält.

15. Druckpaste oder Klotzflotte gemäss Anspruch 14, dadurch gekennzeichnet, dass sie 0,1–10 Gew.-% Verdicker, bezogen auf das Gesamtgewicht, enthält.

16. Druckpaste oder Klotzflotte gemäss Anspruch 14, dadurch gekennzeichnet, dass sie 0,1–5 Gew.-%, vorzugsweise 0,5–1 Gew.-% Antioxidans, bezogen auf den Lactam/Polyalkylenglykol-Komplex, enthält.

**Claims**

1. A process for printing or pad-dyeing textile material made from cellulose fibres in admixture with synthetic fibres, using aqueous printing pastes or padding liquors, by impregnating the textile material, either simultaneously or successively, with an aqueous dispersion of at least one dye difficultly soluble or insoluble in water, the dispersion optionally containing, as further additives: antioxidants, wetting agents, antifoaming agents, softening agents, agents for imparting a flameproof finisch, dirt-, water- and oil-repelling agents, natural or synthetic thickeners and/or an acid, and an aqueous solution of an auxiliary; and subsequently subjecting the printing or the dyeing to a heat treatment, characterised in that there is used as an auxiliary
a complex formed from a lactam of the formula I

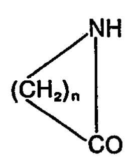 (I),

wherein n is an integer from 3 to 6, and a polyalkylene glycol of the formula II

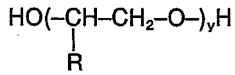 (II)

wherein R is hydrogen or a methyl group, and y is an integer from 5 to 10.

2. A process according to claim 1, characterised in that the auxiliary used is a 1 : 1 molar complex formed from ε-caprolactam and a polyalkylene glycol of the formula II, wherein R and y have the meanings defined in claim 1.

3. A process according to claim 1, characterised in that 0.1 to 5% by weight, preferably 0.5 to 1% by weight, relative to the lactam/polyalkylene glycol complex, of antioxidants are used.

4. A process according to claim 3, characterised in that the antioxidant used is gallic acid propyl ester.

5. A process according to claims 1 to 4, characterised in that the lactam/polyalkylene glycol complex is used in an amount of 5 to 30% by weight, preferably 10 to 20% by weight, relative to the weight of the printing paste or padding liquor.

6. A process according to claim 1, characterised in that there is used the 1 : 1 molar complex formed from ε-caprolactam and polypropylene glycol having a molecular weight of between 300 and 500, preferably about 400, which complex contains 0.5 to 1% by weight of gallic acid propyl ester.

7. A process according to claims 1 to 6, characterised in that the auxiliary is contained in the aqueous printing paste or padding liquor.

8. A process according to claim 1, characterised in that textile material made from cellulose fibres in admixture with polyester fibres is dyed or printed.

9. A process according to claim 1, characterised in that the impregnated or printed material is dried at 80 °C to 130 °C, and is subsequently subjected to the action of heat at 150 °C to 225 °C.

10. A printing paste or padding liquor for carrying out the process according to claims 1 and 7, which contains water, a dye difficultly soluble or insoluble in water, and an auxiliary, as well as optionally, as further additives, antioxidants, wetting agents, antifoaming agents, softeners, additives for imparting a flameproof finish, dirt-, water- and oil-repelling agents, natural or synthetic thickeners and/or an acid, characterised in that there is used as auxiliary a complex formed from a lactam of the formula I

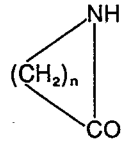 (I),

wherein n is an integer from 3 to 6, and a polyalkylene glycol of the formula II

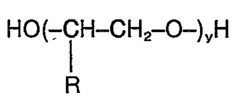 (II)

wherein R is hydrogen or a methyl group, and y is an integer from 5 to 10.

11. A printing paste of padding liquor according to claim 10, characterised in that it contains, as auxiliary, a 1 : 1 molar complex formed from ε-caprolactam and a polyalkylene glycol of the formula II, wherein R and y have the meanings defined in claim 1.

12. A printing paste or padding liquor according to claims 10 and 11, characterised in that it contains 45 to 95% by weight of water, 0.1 to 10% by weight of dye, 5 to 30% by weight of the lactam/polyalkylene glycol complex, and 0.1 to 15% by weight, relative to the total weight, of further additives.

13. A printing paste or padding liquor according to claim 12, characterised in that it contains 60 to 80% by weight of water, 1 to 8% by weight of dye, 10 to 25% by weight of the lactam/polyalkylene glycol complex, and 0.2 to 8% by weight of further additives.

14. A printing paste or padding liquor according to claims 10 to 13, characterised in that it contains a thickener and/or an antioxidant as further additives.

15. A printing paste or padding liquor according to claim 14, characterised in that it contains 0.1 to 10% by weight of thickener, relative to the total weight.

16. A printing paste or padding liquor according to claim 14, characterised in that it contains 0.1 to 5% by weight, preferably 0.5 to 1% by weight, of antioxidant, relative to the lactam/polyalkylene glycol complex.

**Revendications**

1. Procédé d'impression ou de teinture par foulardage de matières textiles en fibres cellulosiques en mélange avec des fibres synthétiques, avec des pâtes d'impression ou bains de foulardage aqueux, selon lequel on imprègne la matière textile, simultanément ou successivement, avec une dispersion aqueuse d'au moins un colorant peu soluble ou insoluble qui contient comme autres additifs, éventuellement, des anti-oxydants, des agents mouillants, des agents anti-moussants, des plastifiants, des additifs pour l'apprêtage ignifugeant, des agents anti-salissures, hydrophobes et oléophobes, des épaississants naturels ou synthétiques et/ou un acide, et une solution aqueuse d'un adjuvant et, consécutivement à l'impression ou la teinture, on soumet à un traitement à la chaleur, caractérisé par le fait que l'on utilise comme adjuvant
un complexe d'un lactame de formule I

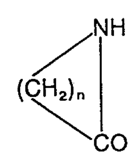 (I)

dans laquelle n désigne un nombre entier de 3 à 6 et d'un polyalkylèneglycol de formule II

$$HO(-\underset{\displaystyle R}{\underset{|}{C}}H-CH_2-O-)_yH \qquad (II)$$

dans laquelle R désigne un atome d'hydrogène ou un groupe méthyle et y désigne un nombre entier de 5 à 10.

2. Procédé selon la revendication 1, caractérisé par le fait que l'on utilise comme adjuvant un complexe molaire 1 : 1 de ε-caprolactame et d'un polyalkylèneglycol de formule II dans laquelle R et y sont définis comme spécifié dans la revendication 1.

3. Procédé selon la revendication 1 caractérisé par le fait que l'on utilise 0,1 à 5% en poids de préférence 0,5, à 1% en poids, par rapport au complexe de lactame/polyoxyalkylèneglycol, d'anti-oxydants.

4. Procédé selon la revendication 3, caractérisé par le fait que l'on utilise comme anti-oxydant le gallate de propyle.

5. Procédé selon les revendications 1 à 4, caractérisé par le fait que l'on utilise le complexe de lactame/polyoxyalkylèneglycol en une quantité de 5 à 30% en poids, de préférence 10 à 20% en poids par rapport au poids de la pâte d'impression ou du bain de foulardage.

6. Procédé selon la revendication 1, caractérisé par le fait que l'on utilise le complexe molaire 1 : 1 de ε-caprolactame et de polypropylèneglycol ayant un poids moléculaire compris entre 300 et 500, de préférence d'environ 400, contenant 0,5 à 1% en poids de gallate de propyle.

7. Procédé selon les revendications 1 à 6, caractérisé par le fait que l'adjuvant est contenu dans la pâte d'impression aqueuse ou le bain de foulardage aqueux.

8. Procédé selon la revendication 1, caractérisé par le fait que l'on teint ou imprime une matière textile en fibre cellulosique en mélange avec des fibres de polyester.

9. Procédé selon la revendication 1, caractérisé par le fait que l'on sèche à 80–130 °C la matière imprégnée ou imprimée et qu'on la soumet consécutivement à l'action de la chaleur de 150 °C à 225 °C.

10. Pâte d'impression ou bain de foulardage pour la mise en œuvre du procédé selon les revendications 1 et 7, contenant de l'eau, un colorant peu soluble ou insoluble dans l'eau, un adjuvant ainsi que comme autres additifs, éventuellement, des anti-oxydants, des agents mouillants, des agents anti-moussants, des agents plastifiants, des additifs pour l'apprêtage ignifugeant, des agents anti-salissures, hydrophobes et oléophobes, des épaississants naturels ou synthétiques et/ou un acide, caractérisé par le fait qu'il utilise comme adjuvant un complexe d'un lactame de formule I

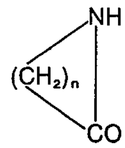

(I)

dans laquelle n désigne un nombre entier de 3 à 6 et d'un polyalkylèneglycol de formule II

$$HO(-\underset{\displaystyle R}{\underset{|}{C}}H-CH_2-O-)_yH \qquad (II)$$

dans laquelle R désigne un atome d'hydrogène ou un groupe méthyle et y désigne un nombre entier de 5 à 10.

11. Pâte d'impression ou bain de foulardage selon la revendication 10, caractérisée par le fait qu'il contient comme adjuvant un complexe molaire 1 : 1 de ε-caprolactame et d'un polyalkylèneglycol de formule II, dans laquelle R et y sont définis comme spécifiés dans la revendication 1.

12. Pâte d'impression ou bain de foulardage selon les revendications 10 et 11, caractérisé par le fait qu'il contient 45 à 95% en poids d'eau, 0,1 à 10% en poids du colorant, 5 à 30% en poids du complexe lactame/polyalkylèneglycol et 0,1 à 15% en poids par rapport au poids total, d'autres additifs.

13. Pâte d'impression ou bain de foulardage selon la revendication 12, caractérisé par le fait qu'il contient 60 à 80% en poids d'eau, 1 à 8% en poids du colorant, 10 à 25% en poids du complexe lactame/polyalkylèneglycol et 0,2 à 8% en poids d'autres additifs.

14. Pâte d'impression ou bain de foulardage selon les revendications 10 à 13, caractérisé par le fait qu'il contient comme autres additifs un épaississant et/ou un anti-oxydant.

15. Pâte d'impression ou bain de foulardage selon la revendication 14, caractérisé par le fait qu'il contient 0,1 à 10% en poids d'épaississant par rapport au poids total.

16. Pâte d'impression ou bain de foulardage selon la revendication 14, caractérisé par le fait qu'il contient 0,1 à 5% en poids, de préférence 0,5 à 1% en poids d'anti-oxydant, par rapport au complexe de lactame/polyoxalkylèneglycol.